(19) Europäisches Patentamt / European Patent Office / Office européen des brevets

(11) **EP 3 833 240 B1**

(12) **EUROPEAN PATENT SPECIFICATION**

(45) Date of publication and mention of the grant of the patent:
**15.05.2024 Bulletin 2024/20**

(21) Application number: **19748802.6**

(22) Date of filing: **30.07.2019**

(51) International Patent Classification (IPC):
**A61B 5/00** *(2006.01)*   **G16H 50/20** *(2018.01)*

(52) Cooperative Patent Classification (CPC):
**A61B 5/746; A61B 5/7264; G16H 10/60; G16H 50/20**

(86) International application number:
**PCT/EP2019/070557**

(87) International publication number:
**WO 2020/030480 (13.02.2020 Gazette 2020/07)**

(54) **INCORPORATING CONTEXTUAL DATA IN A CLINICAL ASSESSMENT**

AUFNAHME VON KONTEXTUELLEN DATEN IN EINE KLINISCHE BEWERTUNG

INCORPORATION DE DONNÉES CONTEXTUELLES DANS UNE ÉVALUATION CLINIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **08.08.2018 US 201862715825 P**

(43) Date of publication of application:
**16.06.2021 Bulletin 2021/24**

(73) Proprietor: **Koninklijke Philips N.V.**
**5656 AG Eindhoven (NL)**

(72) Inventors:
• **ZHAO, Claire, Yunzhu**
  **5656 AE Eindhoven (NL)**
• **XU, Minnan**
  **5656 AE Eindhoven (NL)**
• **CONROY, Bryan**
  **5656 AE Eindhoven (NL)**

(74) Representative: **Philips Intellectual Property & Standards**
**High Tech Campus 52**
**5656 AG Eindhoven (NL)**

(56) References cited:
WO-A1-2015/173675   WO-A1-2018/099767
WO-A1-2018/102821   US-A1- 2014 155 763
US-A1- 2016 302 671   US-A1- 2017 098 037

## Description

TECHNICAL FIELD

Embodiments described herein generally relate to systems and methods for improving patient care and, more particularly but not exclusively, to systems and methods for managing alerts during at least one of patient evaluation, diagnosis, and treatment.

BACKGROUND

Contextual and circumstantial information regarding a patient before, during, or after a clinical encounter are important contributors to clinical support decisions. However, this type of information is subjective in nature, and is usually not measured by clinical measurement devices. Accordingly, this information is often not recorded or otherwise stored in electronic databases.

Additionally, alerting a clinician to consider this type of information too often may overburden the clinician and lead to alert fatigue. This may cause a clinician to develop a reduced sensitivity to alarms and the inclination to act upon them. This in turn could have an adverse impact on the quality of care that a patient receives.

A need exists, therefore, for systems and methods that overcome the disadvantages of existing measurement and alerting techniques.

US 2014/0155763 A1 discloses a computerized method for diagnosing a patient. The method includes selecting a diagnosis and a diagnosis confidence factor for the diagnosis, and determining whether the diagnosis is ambiguous or unambiguous. If the diagnosis is ambiguous, a determination is made as to whether additional testing would produce an unambiguous diagnosis. If so, additional tests are run; if not, a determination is made as to whether a specialist is required, and the diagnosis is referred to either a specialist or a physician according to the determination made.

WO 2015/173675 A1 discloses a patient stratification method and system based on case difficulty. The method includes generating a stratification score for each patient case, which indicates the predicted difficulty of the case, and outputting a patient list ranked according to stratification scores. The patient list may include alerts: for example, a case that is determined to be particularly difficult may be flagged for automatic double reading, that is, a recommendation that two physicians review the case.

US 2016/0302671 A1 discloses a system for classifying a patient's health status as healthy, ambiguous, or sick. The classification is sent to an end-user, such as a nurse or physician.

Other relevant prior art is disclosed in US 2017/098037 A1, WO 2018/099767 A1, WO 2018/102821 A1.

SUMMARY

This summary is provided to introduce a selection of concepts in a simplified form that are further described below in the Detailed Description section. This summary is not intended to identify or exclude key features or essential features of the claimed subject matter, nor is it intended to be used as an aid in determining the scope of the claimed subject matter. The invention is defined by the claims.

According to one aspect, embodiments relate to a method for managing alerts. The method includes receiving patient data using an interface; receiving, using the interface, a classification decision from a classifier executing a classification model, wherein the classification decision is based on the patient data; determining, using a processor executing instructions stored on a memory, whether least one alert filter to the patient data upon determining the classification decision is a borderline classification decision, wherein the at least one alert filter is applied to determine whether to issue a contextual data alert to a clinician to prompt the clinician to consider contextual data related to the patient.

In some embodiments, the method further includes issuing a contextual data alert to the clinician to prompt the clinician to consider contextual data related to the patient.

In some embodiments, determining whether the classification decision is a borderline classification decision includes comparing the classification decision to a decision boundary, determining whether the classification decision is within a predetermined threshold from the decision boundary, and determining the classification decision is a borderline classification decision upon determining the classification decision is within the predetermined threshold from the decision boundary.

In some embodiments, applying the at least one alert filter includes analyzing the patient data to determine whether at least one alert filter condition contradicts the received borderline classification decision, and wherein the method further includes issuing a contextual data alert to the clinician upon the processor determining an alert filter condition contradicts the received borderline classification decision. In some embodiments, the at least one alert filter condition relates to at least one of patient visitation history, patient response to a treatment, a pattern of a clinically measured feature, a type of clinical measurement, and a change in a measured clinical feature.

In some embodiments, the at least one alert filter is selected based on the classification model.

In some embodiments, the contextual data related to the patient includes at least one of mood of the patient, physical appearance of the patient, emotional state of the patient, and agility of the patient.

According to another aspect, embodiments relate to a system for managing alerts. The system includes an interface for receiving patient data and a classification decision from a classifier executing a classification model, wherein the classification decision is based on the patient data; and a processor executing instructions stored on a memory to determine whether the classification decision is a borderline classification decision and apply at least one alert filter to the patient data upon determining the classification decision is a borderline classification decision, wherein the at least one alert filter is applied to determine whether to issue a contextual data alert to a clinician to prompt the clinician to consider contextual data related to the patient.

In some embodiments, the processor is further configured to issue, using a user interface, a contextual data alert to the clinician to prompt the clinician to consider contextual data related to the patient.

In some embodiments, the processor is further configured to determine whether the classification decision is a borderline classification decision by comparing the classification decision to a decision boundary, determining whether the classification decision is within a predetermined threshold from the decision boundary, and determining the classification decision is a borderline classification decision upon determining the classification decision is within a predetermined threshold from the decision boundary.

In some embodiments, the processor applies the at least one alert filter by analyzing the patient data to determine whether at least one alert filter condition contradicts the received borderline classification decision, and the processor is further configured to issue, using a user interface, a contextual data alert to the clinician upon determining an alert filter condition contradicts the received borderline classification decision. In some embodiments, the at least one alert filter condition relates to at least one of patient visitation history, patient response to a treatment, a pattern of a clinically measured feature, a type of clinical measurement, and a change in a measured clinical feature.

In some embodiments, the at least one alert filter is selected based on the classification model.

In some embodiments, the contextual data related to the patient includes at least one of mood of the patient, physical appearance of the patient, emotional state of the patient, and agility of the patient.

According to yet another aspect, embodiments relate to a non-transitory computer readable medium containing computer-executable instructions for performing a method for managing alerts during at least one of patient evaluation, diagnosis and treatment. The medium includes computer-executable instructions for receiving patient data using an interface, computer-executable instructions for receiving, using the interface, a classification decision from a classifier executing a classification model, wherein the classification decision is based on the patient data, computer-executable instructions for determining, using a processor executing instructions stored on a memory, whether the classification decision is a borderline classification decision, and computer-executable instructions for applying, using the processor, at least one alert filter to the patient data upon determining the classification decision is a borderline classification decision, wherein the at least one alert filter is applied to determine whether to issue a contextual data alert to a clinician to prompt the clinician to consider contextual data related to the patient.

BRIEF DESCRIPTION OF DRAWINGS

Non-limiting and non-exhaustive embodiments herein are described with reference to the following figures, wherein like reference numerals refer to like parts throughout the various views unless otherwise specified.

FIG. 1 illustrates a system for managing alerts during at least one of patient evaluation, diagnosis, and treatment in accordance with one embodiment;
FIG. 2 illustrates a workflow of an alert triggering mechanism in accordance with one embodiment;
FIG. 3 depicts a sigmoid function presenting a decision boundary in accordance with one embodiment;
FIG. 4 depicts a flowchart of a method for managing alerts;
FIG. 5 illustrates a graph for quantifying the temporal dynamics of past patient data in accordance with one embodiment; and
FIG. 6 depicts a flowchart of another method for managing alerts.

DETAILED DESCRIPTION

Various embodiments are described more fully below with reference to the accompanying drawings, which form a part hereof, and which show specific exemplary embodiments. However, the concepts of the present disclosure may be implemented in many different forms and should not be construed as limited to the embodiments set forth herein; rather, these embodiments are provided as part of a thorough and complete disclosure, to fully convey the scope of the concepts, techniques and implementations of the present disclosure to those skilled in the art. Embodiments may be practiced as methods, systems or devices. Accordingly, embodiments may take the form of a hardware implementation, an entirely software implementation or an implementation combining software and hardware aspects. The following detailed description is, therefore, not to be taken in a limiting sense.

Reference in the specification to "one embodiment" or to "an embodiment" means that a particular feature, structure, or characteristic described in connection with the embodiments is included in at least one example implementation or technique in accordance with the present disclosure. The appearances of the phrase "in one embodiment" in various places in the specification are not necessarily all referring to the same embodiment. The appearances of the phrase "in some embodiments" in various places in the specification are not necessarily all referring to the same embodiments.

Some portions of the description that follow are presented in terms of symbolic representations of operations on non-transient signals stored within a computer memory. These descriptions and representations are used by those skilled in the data processing arts to most effectively convey the substance of their work to others skilled in the art. Such operations typically require physical manipulations of physical quantities. Usually, though not necessarily, these quantities take the form of electrical, magnetic or optical signals capable of being stored, transferred, combined, compared and otherwise manipulated. It is convenient at times, principally for reasons of common usage, to refer to these signals as bits, values, elements, symbols, characters, terms, numbers, or the like. Furthermore, it is also convenient at times, to refer to certain arrangements of steps requiring physical manipulations of physical quantities as modules or code devices, without loss of generality.

However, all of these and similar terms are to be associated with the appropriate physical quantities and are merely convenient labels applied to these quantities. Unless specifically stated otherwise as apparent from the following discussion, it is appreciated that throughout the description, discussions utilizing terms such as "processing" or "computing" or "calculating" or "determining" or "displaying" or the like, refer to the action and processes of a computer system, or similar electronic computing device, that manipulates and transforms data represented as physical (electronic) quantities within the computer system memories or registers or other such information storage, transmission or display devices. Portions of the present disclosure include processes and instructions that may be embodied in software, firmware or hardware, and when embodied in software, may be downloaded to reside on and be operated from different platforms used by a variety of operating systems.

The present disclosure also relates to an apparatus for performing the operations herein. This apparatus may be specially constructed for the required purposes, or it may comprise a general-purpose computer selectively activated or reconfigured by a computer program stored in the computer. Such a computer program may be stored in a computer readable storage medium, such as, but is not limited to, any type of disk including floppy disks, optical disks, CD-ROMs, magnetic-optical disks, read-only memories (ROMs), random access memories (RAMs), EPROMs, EEPROMs, magnetic or optical cards, application specific integrated circuits (ASICs), or any type of media suitable for storing electronic instructions, and each may be coupled to a computer system bus. Furthermore, the computers referred to in the specification may include a single processor or may be architectures employing multiple processor designs for increased computing capability.

The processes and displays presented herein are not inherently related to any particular computer or other apparatus. Various general-purpose systems may also be used with programs in accordance with the teachings herein, or it may prove convenient to construct more specialized apparatus to perform one or more method steps. The structure for a variety of these systems is discussed in the description below. In addition, any particular programming language that is sufficient for achieving the techniques and implementations of the present disclosure may be used. A variety of programming languages may be used to implement the present disclosure as discussed herein.

In addition, the language used in the specification has been principally selected for readability and instructional purposes and may not have been selected to delineate or circumscribe the disclosed subject matter. Accordingly, the present disclosure is intended to be illustrative, and not limiting, of the scope of the concepts discussed herein.

Embodiments described herein provide systems and methods for considering contextual and/or circumstantial (for simplicity, "contextual") information related to a patient during at least one of evaluation, diagnosis, and/or treatment of the patient. For example, features such as the patient's mood, the patient's general appearance, how the patient moves and walks (e.g., whether they need support), and secondary responses to clinical encounters may be helpful in providing patient care.

However, this information is subjective in nature and not readily or automatically measured by sensor devices. Accordingly, this information is often not considered by machine learning procedures in providing classification decisions regarding a patient.

Classification models may therefore not have or otherwise consider all relevant information to make a confident classification decision regarding a patient. As a result, a classification model may output a "borderline" classification decision indicating that an accurate classification is difficult to achieve (e.g., because contextual data was not gathered/stored in a digital form and therefore was not considered). These borderline classification decisions may ultimately result in false positives and false negatives, and the patient being mistreated.

Accordingly, clinical care systems and methods can improve by considering contextual information related to a patient in these borderline situations. In other words, a clinician's decision may be more accurate if contextual information were considered as opposed to a clinician relying solely on the borderline classification decision.

Features of the present application may therefore alert clinicians of these borderline cases and prompt the clinician to further consider certain contextual information related to the patient. This information may come in the form of a clinician's inputs, and therefore the systems and methods may leverage the clinician's clinical experience to provide insights in addition to those provided by the classification model(s).

Features of the various embodiments described herein, however, also consider the undesirable potential to overwhelm the clinician with excessive alerts. Excess alerts may lead to alert fatigue in which clinicians develop a reduced sensitivity to alerts and the motivation to act upon them. In fact, issues associated with alerts have been ranked as a top technology health hazard.

Furthermore, alerts are often accompanied by sounds that can have a detrimental effect on patient comfort (e.g., by causing heightened stress, delirium, etc.). This is particularly true with vulnerable patients, such as infants or patients in intensive care units.

Accordingly, the features of various embodiments described herein may first consider whether a classification decision is a borderline classification decision. If so, one or more alert filters may be applied to patient data to determine one or more alert filter conditions. If an alert filter condition goes against or otherwise contradicts the borderline classification decision, then a contextual data alert may be issued to a clinician to prompt the clinician to consider certain contextual data related to the patient. If the alert condition agrees with or otherwise does not contradict the classification decision, no contextual data alert will be issued.

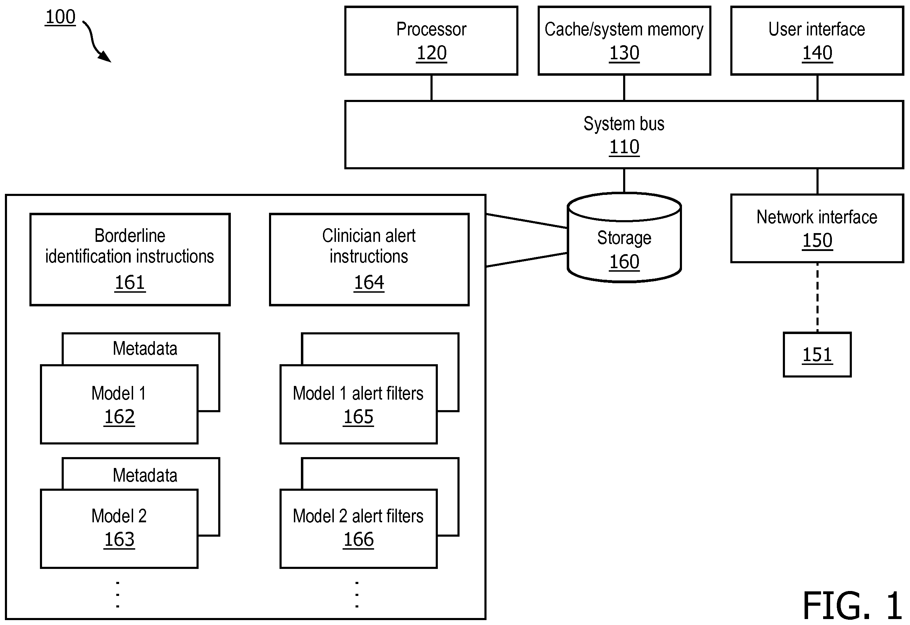

FIG. 1 illustrates a system 100 for managing alerts during at least one of patient evaluation, diagnosis, and treatment. The system 100 may include a processor 120, memory 130, a user interface 140, a network interface 150, and storage 160 interconnected via one or more system buses 110. It will be understood that FIG. 1 constitutes, in some respects, an abstraction and that the actual organization of the system 100 and the components thereof may differ from what is illustrated.

The processor 120 may be any hardware device capable of executing instructions stored on memory 130 or storage 160 or otherwise capable of processing data. As such, the processor 120 may include a microprocessor, field programmable gate array (FPGA), application-specific integrated circuit (ASIC), or other similar devices.

The memory 130 may include various memories such as, for example L1, L2, or L3 cache or system memory. As such, the memory 130 may include static random access memory (SRAM), dynamic RAM (DRAM), flash memory, read only memory (ROM), or other similar memory devices. The exact configuration of the memory 130 may vary as long as instructions for managing alerts can be executed.

The user interface 140 may execute on one or more devices for enabling communication with a user such as a clinician or other type of medical personnel. For example, the user interface 140 may include a display, a mouse, and a keyboard for receiving user commands. In some embodiments, the user interface 140 may include a command line interface or graphical user interface that may be presented to a remote terminal via the network interface 150.

The user interface 140 may execute on a user device such as a PC, laptop, tablet, mobile device, smartwatch, or the like. The exact configuration of the user interface 140 and the device on which it executes may vary as along as the features of various embodiments described herein may be accomplished.

The network interface 150 may include one or more devices for enabling communication with other hardware devices. For example, the network interface 150 may include a network interface card (NIC) configured to communicate according to the Ethernet protocol. Additionally, the network interface 150 may implement a TCP/IP stack for communication according to the TCP/IP protocols. Various alternative or additional hardware or configurations for the network interface 150 will be apparent.

The network interface 150 may be in operable communication with one or more sensor devices 151. In the healthcare context, these may include sensors configured as part of patient monitoring devices that gather various types of information regather patient's health.

The type of sensor devices 151 used may of course vary and may depend on the patient and context. Accordingly, any type of sensor device 151 may be used as long as they can gather or otherwise obtain the required data regarding the entity under analysis.

The sensor device(s) 151 may be in communication with the system 100 over one or more networks that may link the various components with various types of network connections. The network(s) may be comprised of, or may interface to, any one or more of the Internet, an intranet, a Personal Area Network (PAN), a Local Area Network (LAN), a Wide Area Network (WAN), a Metropolitan Area Network (MAN), a storage area network (SAN), a frame relay connection, an Advanced Intelligent Network (AIN) connection, a synchronous optical network (SONET) connection, a digital T1, T3, E1, or E3 line, a Digital Data Service (DDS) connection, a Digital Subscriber Line (DSL) connection, an Ethernet connection, an Integrated Services Digital Network (ISDN) line, a dial-up port such as a V.90, a V.34, or a V.34bis analog modem connection, a cable modem, an Asynchronous Transfer Mode (ATM) connection, a Fiber Distributed Data Interface (FDDI) connection, a Copper Distributed Data Interface (CDDI) connection, or an optical/DWDM network.

The network or networks may also comprise, include, or interface to any one or more of a Wireless Application Protocol (WAP) link, a Wi-Fi link, a microwave link, a General Packet Radio Service (GPRS) link, a Global System for Mobile Communication G(SM) link, a Code Division Multiple Access (CDMA) link, or a Time Division Multiple access (TDMA) link such as a cellular phone channel, a Global Positioning System (GPS) link, a cellular digital packet data (CDPD) link, a Research in Motion, Limited (RIM) duplex paging type device, a Bluetooth radio link, or an IEEE 802.1 1-based link.

The storage 160 may include one or more machine-readable storage media such as read-only memory (ROM), random-access memory (RAM), magnetic disk storage media, optical storage media, flash-memory devices, or similar storage media. In various embodiments, the storage 160 may store instructions for execution by the processor 120 or data upon which the processor 120 may operate. For example, the storage 160 may include borderline classification identification instructions 161, one or more classification models 162 and 163, clinician alert instructions 164, and one or more alert filters 165 and 166 applicable to one or more classification models.

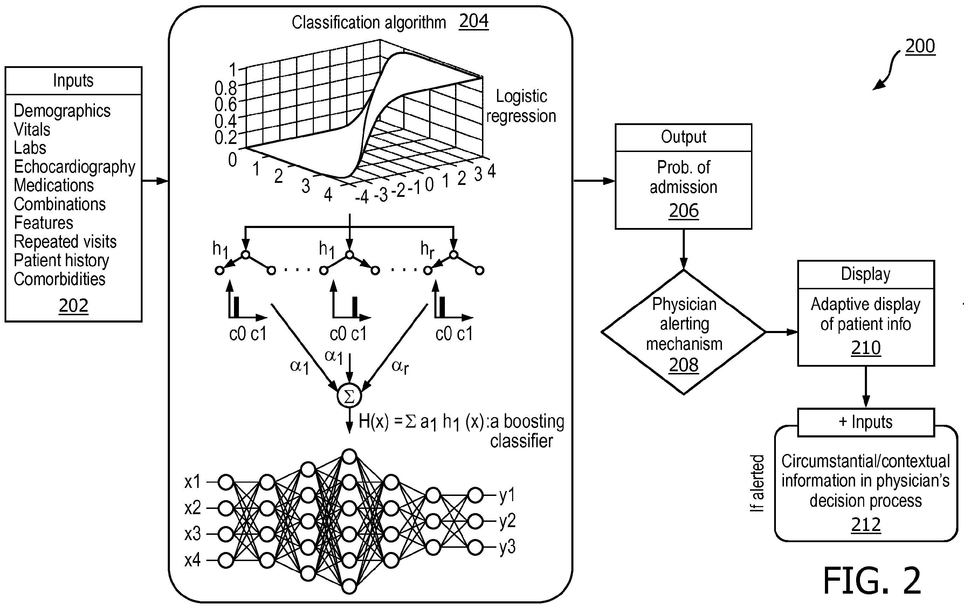

FIG. 2 illustrates a workflow 200 of an alert triggering mechanism in accordance with one embodiment. Step 202 involves receiving one or more types of patient data. This patient data may include, but is not limited to, at least one of patient demographics, patient vitals, patient labs, patient echocardiography data, patient medications, patient visitation history, patient response to treatment(s), patterns of a clinically measured feature, types and/or methods of clinical measurements, changes in a measured clinical feature, or the like. The above list is non-exhaustive, and other types of patient data may be considered to accomplish the features of various embodiments described herein.

In step 204, one or more classification models (such as the models 162 and 163) may analyze the patient data of step 202 to obtain a classification decision. The classification decision may relate to whether a patient tests positive for a condition, whether the patient should be discharged or admitted into a medical unit, or whether the patient should be prescribed certain medication, for example.

The one or more models may execute any type of suitable machine learning classification procedure. These may include, but are not limited to, logistic regression, random forests, neural networks, or the like.

Step 206 may involve receiving a classification decision from the classification model(s). In some embodiments, the output may include a probability of whether or not a patient should be admitted to a certain healthcare unit or the probability that a patient has some condition, for example.

Step 208 involves analyzing the classification decision to determine whether the classification decision is a borderline classification decision. This step may be performed by a physician/clinician alerting mechanism that executes the borderline identification instructions 161 of FIG. 1.

The borderline identification instructions 161 may determine whether a classification decision is a borderline classification decision in accordance with the following. If the classification decision of the current encounter $p_i$ is within a threshold $p_{thresh}$ of a decision boundary $p_b$, then the classification decision is a borderline classification decision. In other words, the borderline classification instructions 161 determine that a classification decision is a borderline classification upon determining that:

$$|p_i - p_b| < p_{thresh}$$

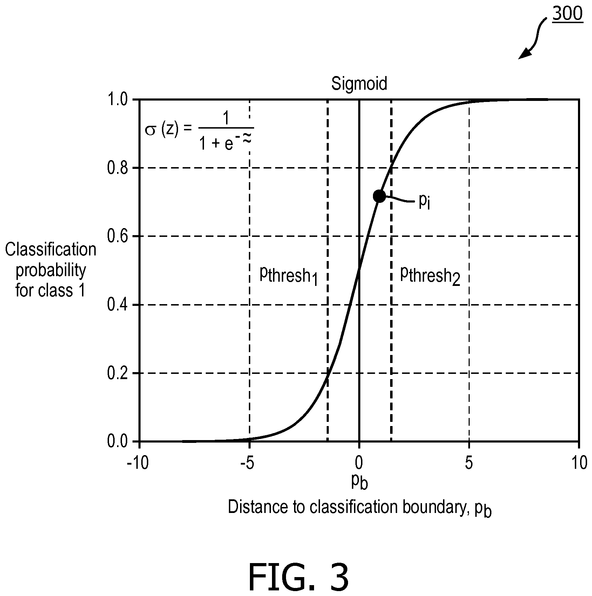

These boundaries may be determined and/or presented in a number of ways. For example, FIG. 3 presents a sigmoid function 300 that presents a decision boundary $p_b$, which is determined previously during training of each classification procedure. For example, the decision boundary $p_b$ may be learned such that values above the boundary result in a positive classification (e.g., admit the patient), and values equal to or below the boundary result in a negative classification (e.g., discharge the patient). Also shown in FIG. 3 is the classification decision $p_i$ of the current test case, and the thresholds $p_{thresh1}$ and $p_{thresh2}$, which are user-defined parameters.

As seen in FIG. 3, the classification decision $p_i$ falls within the area between the decision boundary $p_b$ and the threshold $p_{thresh2}$. Accordingly, the analysis shown in FIG. 3 would result in a borderline classification decision. Although FIG. 3 illustrates a sigmoid function, other types of functions or classification methodologies may be used to determine whether a classification decision is a borderline classification decision.

Upon determining the classification decision is a borderline classification decision, the clinician alerting mechanism may in step 208 determine whether any applicable alert filter conditions contradict the classification decision and may present a display of patient information in step 210. If an alert filter condition contradicts the borderline classification decision, the clinician alerting mechanism may issue an alert to prompt the clinician to consider/provide inputs regarding contextual data related to the patient in step 212.

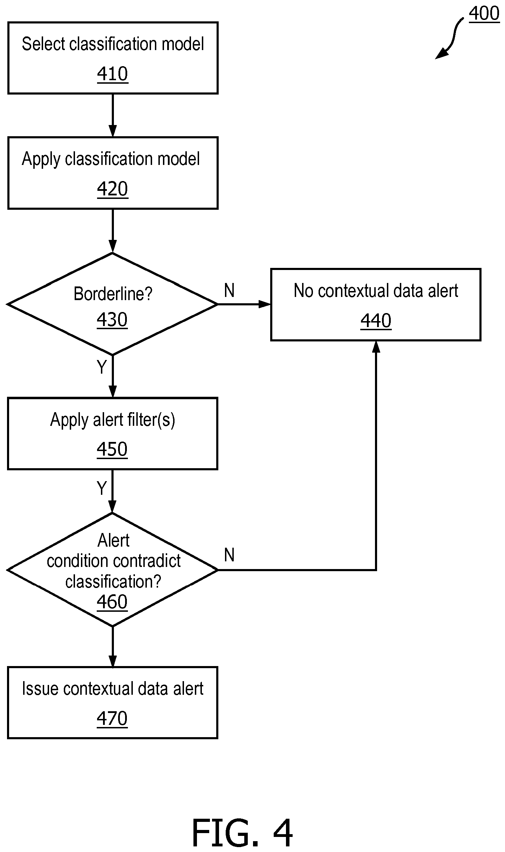

FIG. 4 depicts a flowchart of a method 400 for managing alerts in accordance with one embodiment. Specifically, method 400 includes several steps that may be performed by the clinician alerting mechanism of FIG. 2. Step 410 involves selecting a classification model for use in classifying a patient in accordance with some criteria. The classification model selected in step 410 may include any type of machine learning algorithm, such as logistic regression, random forests, convolutional neural networks (CNNs), recurrent neural networks (RNNs), or the like.

Step 420 involves applying the classification model selected in step 410 to patient data to receive a classification decision regarding the patient. The classification decision may relate to whether the patient should be admitted into the healthcare institution (such as a specific department thereof) or discharged and sent home. Or, in other embodiments, not part of the invention, the classification decision may relate to whether or not the patient is diagnosed with a certain condition. In yet other embodiments, the classification decision may relate to whether or not the patient should be prescribed certain medication. These embodiments are merely exemplary and it is contemplated that the classification model may be configured to make other types of classification decisions.

Step 430 involves determining whether the classification decision is a borderline classification decision. As discussed above, the classification model may not be able to make a classification decision with a required or desired level of confidence.

For example, if the classification model is configured to output a classification in terms of a numeric value (such as on a sigmoid function scale as shown in FIG. 3), the classification decision may be considered a borderline classification if the decision falls within some threshold distance from a decision boundary.

If the answer in step 430 is no (i.e., the classification decision is not a borderline classification decision), the method 400 may proceed to step 440. Step 440 specifies that if the classification decision is not a borderline classification, no contextual data alerts will be issued. This is essentially because there is not a requisite degree of uncertainty and therefore additional contextual data isn't needed to obtain an accurate classification decision.

If the answer in step 430 is yes (i.e., the classification decision is a borderline classification), the method 400 may proceed to step 450. Step 450 involves applying at least one alert filter. The alert filter(s) consider certain types of patient data to obtain a deeper view of the patient's overall state, as well as a clinician's knowledge of the patient's health.

The data subjected to the alert filters may include some of or all of the data upon which the classification is based. Additionally or alternatively, the data subjected to the alert filters may include data that is different than the data upon which the classification decision is based.

In some embodiments, the alert filter may consider patient visitation history. For example, if the patient visits the Emergency Department (ED) for the first time for a given symptom, they are often admitted for a more thorough examination. If the patient visits the ED repeatedly for the same condition(s) and in a short period of time, admitting the patient to the hospital (or a certain department thereof) will help determine if the condition is worsening and the condition's associated causes. On the other hand, if the repeated visits are spaced over a long period of time, efforts may be channeled towards stabilizing the patient in the ED and then sending them home.

In other words, if the classification decision was an instruction to admit the patient, and it is the patient's first visit to the hospital, a clinician would likely want to conduct a more thorough examination. Accordingly, an alert filter may be "is this the patient's first visit?" If the answer is "yes," the method may ultimately be more inclined to issue a contextual data alert to prompt the clinician to input or otherwise consider contextual data related to the patient.

On the other hand, if the classification decision was an instruction to admit the patient but the patient has made multiple visits in the past, the clinician is likely already familiar with the patient. In this scenario, the method 400 may be more inclined to not issue a contextual data alert as this would likely only annoy the clinician. Or, another alert filter may be applied.

Another alert filter may look for patterns of measurements that are indicative of high acuity and therefore warrant admission of the patient to a higher level of care. These patterns may include, but are not limited to, the presence of certain measurements, the frequency of measurement, the method through which a measurement is obtained, or the like.

Certain measurements are only ordered by a clinician when the clinician is already concerned about the patient's condition. Therefore, the mere fact that a certain measurement has been ordered is indicative of the patient's deteriorating state (as well as the clinician's knowledge of the patient's deteriorating state). In these situations, the method 400 may be inclined to not issue a contextual data alert as the clinician is already familiar with the patient and the patient's state.

These features can be discovered by analyzing the features that are less common across a patient population. Additionally, if a measurement (e.g., vitals) is measured too frequently, it is often indicative of the care team's awareness of the patient's deteriorating state. Furthermore, the usage of invasive (as opposed to non-invasive) measurement methods, or higher sensitivity methods, to measure a certain clinical value is usually an indication of higher acuity.

For example, invasively measured blood pressure indicates a higher degree of clinician concern than non-invasive methods. In these instances, alerting the clinician of a borderline admission decision is redundant and should be avoided since the clinician already knows the patient is deteriorating and should be admitted.

Another alert filter may relate to patient data trends. For patients with acute exacerbation originating from an underlying chronic disease, patient history or the way the patient has changed over time is especially important in channeling clinical decisions. Trend analysis of past patient data can therefore provide valuable insight with respect to a patient's condition.

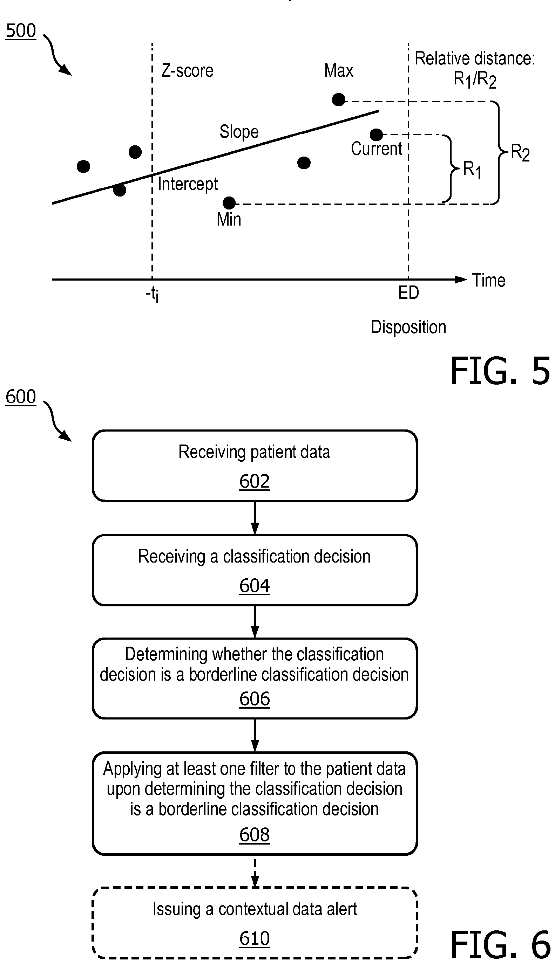

For example, FIG. 5 illustrates a graph 500 used for quantifying the temporal dynamics of past data. Statistics such as the minimum, maximum, median, standard deviation, relative distance of a current data point $x$ to minimum (i.e., $(x - Min) / (Max - Min)$), Z-score of the current data point $x$ are all helpful measures. In addition, the slope and intercept of linear regression through the past data points are also clinically indicative.

It may also be useful to carry out the above analysis over different temporal durations of past data. For instance, all available data may be considered or only data from a certain window is considered such as the past six months, the past month, the past 7 days, data from the past 2 days, the past 24 hours, during triage, etc. If the clinical value being accessed changes by a large extent as indicated by the aforementioned statistics, an alert filter condition may suggest the patient should be admitted.

Another alert filter may consider patient responsiveness to treatment and/or the aggressiveness of treatment provided to the patient in the ED. If a patient is responsive to treatment in the ED and is stabilized, then the clinician should consider possible discharge. If, however, the borderline classification decision was to admit the patient, and the patient has been responsive to treatment and is stabilized, an alarm should be issued to the clinician as the alert filter condition contradicts the classification decision. In other words, if the borderline classification decision is to admit the patient, but other patient data shows that the patient has been responsive to treatment and is stabilized, then an alert should be issued to the clinician prompting the clinician to consider contextual data related to the patient.

For instance, discharge is likely considered if the patient responded well to diuretics administered in the ED. A simple way to evaluate treatment responsiveness may be to estimate dosage and duration of medications. For example, one could calculate equivalent dosages of different types of diuretic drugs with different routes of administration (e.g., per oral drip IV, continuous IV pump, etc.). Escalating dosages generally indicate non-responsiveness and therefore admission to higher levels of care. More comprehensive but complicated assessment may be through analysis of changes in edema and fluid retention on chest X-ray images.

Referring back to FIG. 4, step 460 involves determining whether an alert filter condition contradicts the classification decision from step 420. Filters such as those discussed above may be set or otherwise configured to determine if certain types of patient data satisfy/do not satisfy one or more conditions that have some significance or some implication of a patient's health status. If the one or more alert filter conditions do not contradict the classification decision(s), the method 400 may proceed to step 440 and no contextual data alert will be issued.

For example, the classification decision may have been to admit a patient to the emergency department. The alert filter condition (based on the alert filter(s)), may indicate that the patient has not responded to treatment. In this case, the alert condition may be said to agree with or otherwise not contradict the classification decision. The method 400 may then proceed to step 440 in which case no contextual data alert is issued.

If on the other hand, the alert condition indicates that the patient has responded to treatment, the alert filter condition may be said to contradict the classification decision of admitting the patient to the ED. In this case the method 400 of FIG. 4 may proceed to step 470.

Step 470 involves issuing a contextual data alert to a clinician. The contextual data alert may prompt the clinician to consider certain types of contextual data related to the patient so that the patient can receive more appropriate treatment. As discussed above, this information about the patient is usually unrecorded. However, it can be incorporated in the decision process to resolve difficult cases (i.e., cases with borderline classification decisions).

An alert may be issued to the clinician via any suitable user device. These alerts may include an audio-based alert, a visual-based alert, a haptic-based alert, or some combination thereof. For example, with reference back to FIG. 2, the systems and methods described herein may output, via any suitable interface executing on any suitable user device, an adaptive display of patient information and prompt the clinician to input certain types of contextual information related to the patient.

Specifically, the alert may involve a prompt for the clinician's attention, a presentation of more detailed clinical parameters and their temporal trends, and a presentation of the borderline clinical values. This information may be presented in any suitable forms such as text, graphics, audio messages, graphs, animations, or the like.

The clinician may then consider contextual information regarding the patient and/or the patient encounter that is not observable by the classification model(s). The clinician may enter this contextual information using input devices such as a keyboard, a mouse, a microphone, or by interacting with a touchscreen on a display, scanning a handwritten note to be analyzed by OCR techniques, or the like.

FIG. 6 depicts a flowchart of a method 600 for managing alerts in accordance with one embodiment. Step 602 involves receiving patient data using an interface. The type of patient data received may of course vary and may depend on the reason for the patient's visit to a healthcare institution.

The patient data may be received via an interface such as the interface 150 of FIG. 1. The patient data may be gathered by any suitable sensor devices such as those for measuring patient parameters such as those listed in step 202 of FIG. 2.

Step 604 involves receiving, using the interface, a classification decision from a classifier executing a classification model, wherein the classification decision is based on the patient data. The classification decision may relate to a clinical care decision such as whether to admit the patient into a healthcare institution department or to treat the patient and send them home. The classification decision may be made by any suitable classification model executing one or more various machine learning procedures such as logistic regression, random forests, CNNs, RNNs, or the like.

Step 606 involves determining, using a processor executing instructions stored on a memory, whether the classification decision is a borderline classification decision. Oftentimes a classification decision may not have a desired confidence due to, for example, lack of certain types of patient data.

For example, a classification decision may be within a certain threshold of a decision boundary as described in conjunction with FIG. 3. If the classification decision falls within a threshold distance from the classification decision boundary, it may be referred to as a borderline classification decision.

Step 608 involves applying, using the processor, at least one alert filter to the patient data upon determining the classification decision is a borderline classification decision, wherein the at least one alert filter is applied to determine whether to issue a contextual data alert to a clinician to prompt the clinician to consider contextual data related to the patient. The at least one alert filter may be applied to certain types of patient data to determine one or more alert filter conditions. As discussed above, the alert filter condition(s) may relate to specific aspects regarding a patient's health, their personal history, their medical history, etc. This information may include whether the patient has responded well to previous treatment(s), the type of patient parameters previously measured, how these parameters have been previously measured, or the like.

Step 610 is optional and involves issuing a contextual data alert to the clinician to prompt the clinician to consider contextual data related to the patient. A processor such as the processor 120 of FIG. 1 may analyze the alert filter condition in conjunction with the borderline classification decision to determine whether the alert filter condition contradicts the borderline classification decision. Upon determining the alert filter condition contradicts the classification decision, the processor may issue, via an interface, a contextual data alert to prompt a clinician to consider contextual data related to the patient.

The methods, systems, and devices discussed above are examples. Various configurations may omit, substitute, or add various procedures or components as appropriate. For instance, in alternative configurations, the methods may be performed in an order different from that described, and that various steps may be added, omitted, or combined. Also, features described with respect to certain configurations may be combined in various other configurations. Different aspects and elements of the configurations may be combined in a similar manner. Also, technology evolves and, thus, many of the elements are examples and do not limit the scope of the disclosure or claims.

Embodiments of the present disclosure, for example, are described above with reference to block diagrams and/or operational illustrations of methods, systems, and computer program products according to embodiments of the present disclosure. The functions/acts noted in the blocks may occur out of the order as shown in any flowchart. For example, two blocks shown in succession may in fact be executed substantially concurrent or the blocks may sometimes be executed in the reverse order, depending upon the functionality/acts involved. Additionally, or alternatively, not all of the blocks shown in any flowchart need to be performed and/or executed.

For example, if a given flowchart has five blocks containing functions/acts, it may be the case that only three of the five blocks are performed and/or executed. In this example, any of the three of the five blocks may be performed and/or executed.

A statement that a value exceeds (or is more than) a first threshold value is equivalent to a statement that the value meets or exceeds a second threshold value that is slightly greater than the first threshold value, e.g., the second threshold value being one value higher than the first threshold value in the resolution of a relevant system. A statement that a value is less than (or is within) a first threshold value is equivalent to a statement that the value is less than or equal to a second threshold value that is slightly lower than the first threshold value, e.g., the second threshold value being one value lower than the first threshold value in the resolution of the relevant system.

Specific details are given in the description to provide a thorough understanding of example configurations (including implementations). However, configurations may be practiced without these specific details. For example, well-known circuits, processes, algorithms, structures, and techniques have been shown without unnecessary detail in order to avoid obscuring the configurations. This description provides example configurations only, and does not limit the scope, applicability, or configurations of the claims. Rather, the preceding description of the configurations will provide those skilled in the art with an enabling description for implementing described techniques. Various changes may be made in the function and arrangement of elements without departing from the spirit or scope of the disclosure.

Having described several example configurations, various modifications, alternative constructions, and equivalents may be used without departing from the spirit of the disclosure. For example, the above elements may be components of a larger system, wherein other rules may take precedence over or otherwise modify the application of various implementations or techniques of the present disclosure. Also, a number of steps may be undertaken before, during, or after the above elements are considered.

Having been provided with the description and illustration of the present application, one skilled in the art may envision variations, modifications, and alternate embodiments.

For example, the above-described systems and methods, in an embodiment not being part of the invention, can be applied to emergency department triage of acute heart failure (AHF) patients. ED is the first in line of care for patients suffering from AHF, and a triage decision of "admit to hospital" or "send home" needs to be made quickly.

To alleviate the burden in the ED and the healthcare institution (e.g., a hospital) as a whole, the systems and methods described above may analyze patients suffering from heart failure to recognize those that should be admitted and those who can be treated and sent home. Accordingly, the systems and methods described above may help correctly identifier borderline cases by the upstream machine learning classification algorithm and prompt clinicians, when appropriate, to reconsider the models' classification/triage decision in the context of the current patient and patient encounter. As discussed above, this contextual information is generally not recorded and therefore not considered by the algorithm.

In fact, several types of contextual information contribute to acute exacerbations but are not recorded or stored in electronic databases. The systems and methods of various embodiments described above therefore improves the management of patients with, for example AHF.

In addition to AHF, the systems and methods of various embodiments described above and not part of the invention can be generalized to other conditions, such as kidney disease, asthma, and COPD. Different version of the alert mechanisms may be used in conjunction with various different machine learning procedures. For example, the alert triggers used in conjunction with AHF will likely be different from that used for chronic kidney disease. Additionally, the framework proposed may be adapted to changing clinical practices and variation in clinical practice between clinicians, hospitals, geographical areas, or the like.

The systems and methods of various embodiments described above and not part of the invention can also be generalized to transfer decisions in clinical settings other than the ED. For example, the systems and methods described above may be implemented in the general ward.

In fact, the processes outlined for prompting clinicians for contextual information can be generalized to any classification task. For example, the systems and methods described above may be applied to classification tasks involving financial market analysis, logistic operations, manufacturing, weather analysis, or the like.

Regardless of the exact application, the identification of borderline cases close to a decision boundary can be based on a classification probability of a distance measure (e.g., Euclidean distance). The alert mechanism(s) can be co-developed with the upstream machine learning classification model. Accordingly, specific alert filters may be applied based on the classification model used.

Additionally or alternatively, the alert mechanisms could be used with existing CDS algorithms such as those developed by the Applicant. Additionally, the alert systems and methods can be created using machine learning whose target population is the subpopulation of borderline cases identified by the upstream classification algorithm, thereby building a hierarchical pipeline of algorithms. For instance, alerts may be issued for a candidate case if the associated decision by the algorithm goes against the decision by any one of the conditions specified by the method. More complicated relations between the conditions can be learned if the machine learning is deployed. Lastly, the alert fatigue prevention methods described above may be used for developing new CDS algorithms or refining existing ones.

**Claims**

1. A method for managing alerts during at least one of patient evaluation, diagnosis, and treatment, the method comprising:

receiving patient data using an interface (150); obtaining, at a processor (120), a classification decision from a classifier executing a classification model (162, 163), wherein the classification decision is based on the patient data; determining, using a processor (120) executing instructions (161) stored on a memory (130, 160), whether the classification decision is a borderline classification decision; applying, using the processor (120), at least one alert filter (165, 166) to the patient data upon determining the classification decision is a borderline classification decision, wherein the at least one alert filter (165, 166) is applied to determine whether to issue a contextual data alert to a clinician to prompt the clinician to consider contextual data related to the patient, **characterized in that** applying the at least one alert filter (165, 166) includes analyzing the patient data to determine whether at least one alert filter condition contradicts the obtained borderline classification decision; and further **characterized in that** the method comprises the step of issuing a contextual data alert to the clinician upon the processor (120) determining an alert filter condition contradicts the obtained borderline classification decision.

2. The method of claim 1, wherein determining whether the classification decision is a borderline classification includes:

comparing the classification decision to a decision boundary, determining whether the classification decision is within a predetermined threshold from the decision boundary, and determining the classification decision is a borderline classification decision upon determining the classification decision is within the predetermined threshold from the decision boundary.

3. The method of claim 1, wherein no contextual data alert is issued to the clinician upon the processor (120) determining that no alert filter condition contradicts the obtained borderline classification decision.

4. The method of claim 1 wherein the at least one alert filter condition relates to at least one of patient visitation history, patient response to a treatment, a pattern of a clinically measured feature, a type of clinical measurement, and a change in a measured clinical feature.

5. The method of claim 1 wherein the at least one alert filter (165, 166) is selected based on the classification model (162, 163).

6. The method of claim 1 wherein the contextual data related to the patient includes at least one of mood of the patient, physical appearance of the patient, emotional state of the patient, and agility of the patient.

7. A system (100) for managing alerts during at least one of patient evaluation, diagnosis, and treatment, the system comprising:

an interface (150) for receiving patient data; and a processor (120) executing instructions stored on a memory (130, 160) to:

obtain a classification decision from a classifier executing a classification model (162, 163), wherein the classification decision is based on the patient data, determine whether the classification decision is a borderline classification decision, apply at least one alert filter (165, 166) to the patient data upon determining the classification decision is a borderline classification decision, wherein the at least one alert filter (165, 166) is applied to determine whether to issue a contextual data alert to a clinician to prompt the clinician to consider contextual data related to the patient, **characterized in that** the processor applies the at least one alert filter (165, 166) by analyzing the patient data to determine whether at least one alert filter condition contradicts the obtained borderline classification decision, and issues using a user interface (140), a contextual data alert to the clinician upon determining an alert condition contradicts the obtained borderline classification decision.

8. The system (100) of claim 7 wherein the processor (120) is further configured to determine whether the classification decision is a borderline classification decision by:

comparing the classification decision to a decision boundary, determining whether the classification decision is within a predetermined threshold from the decision boundary, and determining the classification decision is a borderline classification decision upon determining the classification decision is within a predetermined threshold from the decision boundary.

9. The system (100) of claim 7, wherein the processor (120) issues no contextual data alert to the clinician upon determining that no alert filter condition contradicts the obtained borderline classification decision.

10. The system (100) of claim 7 wherein the at least one alert filter condition relates to at least one of patient visitation history, patient response to a treatment, a pattern of a clinically measured feature, a type of clinical measurement, and a change in a measured clinical feature.

11. The system (100) of claim 7 wherein the at least one alert filter is selected based on the classification model.

12. The system (100) of claim 7 wherein the contextual data related to the patient includes at least one of mood of the patient, physical appearance of the patient, emotional state of the patient, and agility of the patient.

13. A non-transitory computer readable medium containing computer-executable instructions for performing a method for managing alerts during at least one patient evaluation, diagnosis, and treatment, the medium comprising:

computer-executable instructions for receiving patient data using an interface (150); computer-executable instructions for obtaining, at a processor (120), a classification decision from a classifier executing a classification model (162, 163), wherein the classification decision is based on the patient data; computer-executable instructions (161) for determining, using a processor (120) executing instructions stored on a memory (130, 160), whether the classification decision is a borderline classification decision; computer-executable instructions for applying, using the processor (120), at least one alert filter (165, 166) to the patient data upon determining the classification decision is a borderline classification decision, wherein the at least one alert filter (165, 166) is applied to determine whether to issue a contextual data alert to a clinician to prompt the clinician to consider contextual data related to the patient, **characterized in that** applying the at least one alert filter (165, 166) includes analyzing the patient data to determine whether at least one alert filter condition contradicts the obtained borderline classification decision; and further **characterized in that** the medium comprises

computer-executable instructions for issuing, using a user interface (140), a contextual data alert to the clinician upon the processor (120) determining an alert filter condition contradicts the obtained borderline classification decision.

## Patentansprüche

1. Verfahren zur Verwaltung von Alarmen während mindestens einer Patientenbewertung, Diagnose und Behandlung, wobei das Verfahren umfasst: Empfang von Patientendaten über eine Schnittstelle (150); Erhalten, an einem Prozessor (120), einer Einstufungsentscheidung von einem Klassifizierer, der ein Einstufungsmodell (162, 163) ausführt, wobei diese Einstufungsentscheidung auf den Patientendaten basiert; Bestimmen, unter Verwendung eines Prozessors (120), welcher Anweisungen (161) ausführt, die in einem Speicher (130, 160) gespeichert sind, ob die Einstufungsentscheidung eine grenzwertige Einstufungsentscheidung ist; Anwenden, unter Verwendung des Prozessors (120), mindestens eines Warnfilters (165, 166) auf die Patientendaten, wenn bestimmt wird, dass die Einstufungsentscheidung eine grenzwertige Einstufungsentscheidung ist, wobei der mindestens eine Warnfilter (165, 166) angewendet wird, um zu bestimmen, ob eine kontextbezogene Datenwarnung an einen Arzt der Klinik ausgegeben werden soll, um den Arzt der Klinik aufzufordern, kontextbezogene Daten in Bezug auf den Patienten zu berücksichtigen, **dadurch gekennzeichnet, dass** die Anwendung des mindestens einen Alarmfilters (165, 166) Folgendes umfasst: Analysieren der Patientendaten, um festzustellen, ob mindestens eine Alarmfilterbedingung der erhaltenen grenzwertigen Einstufungsentscheidung widerspricht; und ferner **dadurch gekennzeichnet, dass** das Verfahren den folgenden Schritt umfasst: Ausgabe eines kontextbezogenen Datenalarms an den Arzt der Klinik, wenn der Prozessor (120) feststellt, dass eine Alarmfilterbedingung der erhaltenen grenzwertigen Einstufungsentscheidung widerspricht.

2. Verfahren nach Anspruch 1, bei dem bestimmt wird, ob die Einstufungs Entscheidung eine grenzwertige Einstufung beinhaltet: Vergleichen der Einstufungsentscheidung mit einer Entscheidungsgrenze, Bestimmen, ob die Einstufungsentscheidung innerhalb eines vorbestimmten Schwellenwertes vor der Entscheidungsgrenze liegt, und Feststellen, dass es sich bei der Einstufungsentscheidung um eine grenzwertige Einstufungsentscheidung handelt, wenn festgestellt wird, dass die Einstufungsentscheidung innerhalb des vorgegebenen Schwellenwerts vor der Entscheidungsgrenze liegt.

3. Verfahren nach Anspruch 1, wobei keine kontextbezogene Datenwarnung an den Arzt der Klinik ausgegeben wird, wenn der Prozessor (120) feststellt, dass keine Alarmfilterbedingung der erhaltenen Entscheidung zur Grenzwert-Einstufung widerspricht.

4. Verfahren nach Anspruch 1, wobei sich die mindestens eine Alarmfilterbedingung auf mindestens eines der folgenden Merkmale bezieht: Historie der Arztvisiten beim Patienten, Reaktion des Patienten auf eine Behandlung, Muster eines klinisch gemessenen Merkmals, Art der klinischen Messung und Änderung eines gemessenen klinischen Merkmals.

5. Verfahren nach Anspruch 1, wobei der mindestens eine Alarmfilter (165, 166) auf Grundlage des Einstufungsmodells (162, 163) ausgewählt ist.

6. Verfahren nach Anspruch 1, bei dem die auf den Patienten bezogenen Kontextdaten mindestens eines der folgenden Elemente umfassen: Stimmung des Patienten, physisches Erscheinungsbild des Patienten, emotionaler Zustand des Patienten und Beweglichkeit des Patienten.

7. System (100) zur Verwaltung von Alarmen während mindestens einer Patientenbewertung, Diagnose und Behandlung, das System umfasst: Eine Schnittstelle (150) zum Empfang von Patientendaten; und einen Prozessor (120), der in einem Speicher (130, 160) gespeicherte Anweisungen ausführt zum: Erhalten einer Einstufungsentscheidung von einem Klassifizierer, der ein Einstufungsmodell (162, 163) ausführt, wobei die Einstufungsentscheidung auf den Patientendaten basiert, Feststellen, ob es sich bei der Einstufungsentscheidung um eine grenzwertige Einstufungsentscheidung handelt, Anwenden mindestens eines Warnfilters (165, 166) auf die Patientendaten, wenn festgestellt wird, dass es sich bei der Einstufungsentscheidung um eine grenzwertige Einstufungsentscheidung handelt, wobei der mindestens eine Warnfilter (165, 166) angewandt wird, um zu bestimmen, ob eine kontextbezogene Datenwarnung an einen Arzt der Klinik ausgegeben werden soll, um den Arzt der Klinik aufzufordern, kontextbezogene Daten in Bezug auf den Patienten zu berücksichtigen, **dadurch gekennzeichnet, dass** der Prozessor den mindestens einen Alarmfilter (165, 166) anwendet, indem er die Patientendaten analysiert, um festzustellen, ob mindestens eine Alarmfilterbedingung der erhaltenen grenzwertige Einstufungsentscheidung widerspricht, und Ausgabe, unter Verwendung einer Benutzerschnittstelle (140), einer kontextbezogenen Datenwarnung an den Arzt der Klinik, wenn festgestellt wird, dass eine Warnbedingung im Widerspruch zu der getroffenen Entscheidung über die Einstufung als Grenzfall steht.

8. System (100) nach Anspruch 7, wobei der Prozessor (120) ferner konfiguriert ist, zu bestimmen, ob es sich bei der Einstufungsentscheidung um eine grenzwertige Einstufungsentscheidung handelt, indem: Die Einstufungsentscheidung mit einer Entscheidungsgrenze verglichen wird, bestimmt wird, ob die Einstufungsentscheidung innerhalb eines vorbestimmten Schwellenwertes vor der Entscheidungsgrenze liegt, und festgestellt wird, dass es sich bei der Einstufungsentscheidung um eine grenzwertige Einstufungsentscheidung handelt, wenn festgestellt wird, dass die Einstufungs-Entscheidung innerhalb eines vorgegebenen Schwellenwerts vor der Entscheidungsgrenze liegt.

9. System (100) nach Anspruch 7, wobei der Prozessor (120) keine kontextbezogenen Datenwarnungen an den Arzt der Klinik ausgibt, wenn er feststellt, dass keine Bedingung des Warnfilters der getroffenen Entscheidung zur Grenzwertigkeit widerspricht.

10. System (100) nach Anspruch 7, wobei die mindestens eine Alarmfilterbedingung sich auf mindestens eines der folgenden Merkmale bezieht: Historie der Arztvisiten beim Patienten, Reaktion des Patienten auf eine Behandlung, Muster eines klinisch gemessenen Merkmals, Art der klinischen Messung und Änderung eines gemessenen klinischen Merkmals.

11. System (100) nach Anspruch 7, wobei der mindestens eine Alarmfilter basierend auf dem Einstufungsmodell ausgewählt wird.

12. System (100) nach Anspruch 7, wobei die Kontextdaten des Patienten mindestens eines der folgenden Elemente umfassen: Stimmung des Patienten, physisches Erscheinungsbild des Patienten, emotionaler Zustand des Patienten und Beweglichkeit des Patienten.

13. Ein nicht nur vorübergehendes computerlesbares Medium, das vom Computer ausführbare Anweisungen zur Durchführung eines Verfahrens zur Verwaltung von Warnungen während mindestens einer Patientenbeurteilung, diagnose und behandlung enthält, wobei das Medium umfasst: Von einem Computer ausführbare Anweisungen für den Empfang von Patientendaten über eine Schnittstelle (150); von einem Computer ausführbare Anweisungen, um in einem Prozessor (120) eine Einstufungsentscheidung von einem Klassifizierer zu erhalten, der ein Einstufungsmodell (162, 163) ausführt, wobei die Einstufungsentscheidung auf den Patientendaten basiert; von einem Computer ausführbare Anweisungen (161) zum Bestimmen, unter Verwendung eines Prozessors (120), der in einem Speicher (130, 160) gespeicherte Anweisungen ausführt, ob die Einstufungsentscheidung eine grenzwertige Einstufungsentscheidung ist; von einem Computer ausführbare Anweisungen zum Anwenden, unter Verwendung des Prozessors (120), mindestens eines Warnfilters (165, 166) auf die Patienten-Daten, sobald bestimmt wird, dass die Einstufungsentscheidung eine grenzwertige Einstufungsentscheidung ist, wobei der mindestens eine Warnfilter (165, 166) angewandt wird, um zu bestimmen, ob eine kontextbezogene Datenwarnung an einen Arzt der Klinik ausgegeben werden soll, um den Arzt der Klinik aufzufordern, kontextbezogene Daten in Bezug auf den Patienten zu berücksichtigen, **dadurch gekennzeichnet, dass** die Anwendung des mindestens einen Filters (165, 166) für Alarmmeldungen das Analysieren der Patientendaten enthält, um zu bestimmen, ob mindestens eine Alarmfilterbedingung der erhaltenen grenzwertigen Einstufungsentscheidung widerspricht; und ferner **dadurch gekennzeichnet, dass** das Medium von einem Computer ausführbare Anweisungen umfasst, um unter Verwendung einer Benutzerschnittstelle (140) eine kontextbezogene Datenwarnung an den Arzt der Klinik auszugeben, wenn der Prozessor (120) feststellt, dass eine Alarmfilterbedingung der erhaltenen grenzwertigen Einstufungsentscheidung widerspricht.

## Revendications

1. Méthode de gestion des alertes au cours d'au moins une évaluation, un diagnostic et un traitement du patient, la méthode consistant à: recevoir des données du patient à l'aide d'une interface (150); obtenir, au niveau d'un processeur (120), une décision de classification de la part d'un classificateur exécutant un modèle de classification (162, 163), la décision de classification étant basée sur les données du patient; déterminer, à l'aide d'un processeur (120) exécutant des instructions (161) stockées dans une mémoire (130, 160), si la décision de classification est une décision de classification limite; appliquer, à l'aide du processeur (120), au moins un filtre d'alerte (165, 166) aux données du patient après avoir déterminé que la décision de classification est une décision de classification limite, dans lequel au moins un filtre d'alerte (165, 166) est appliqué pour déterminer s'il faut émettre une alerte de données contextuelles à un clinicien pour l'inciter à prendre en compte les données contextuelles relatives au patient, **caractérisée par le fait que** l'application d'au moins un filtre d'alerte (165, 166) comprend analyser les données du patient pour déterminer si au moins une condition de filtre d'alerte contredit la décision de classification limite obtenue; et **caractérisé en outre en ce que** la méthode comprend l'étape consistant à émettre une alerte de données contextuelles au clinicien lorsque le processeur (120) détermine qu'une condition de filtre d'alerte contredit la décision de classification limite obtenue.

2. Méthode selon la revendication 1, dans laquelle le fait de déterminer si la décision de classification est une classification limite comprend: comparer la décision de classification à une limite de décision, déterminer si la décision de classification se situe à l'intérieur d'un seuil prédéterminé par rapport à la limite de décision, et déterminer que la décision de classification est une décision de classification limite après avoir déterminé que la décision de classification se situe à l'intérieur du seuil prédéterminé de la limite de décision.

3. Méthode selon la revendication 1, dans laquelle aucune alerte de données contextuelles n'est émise à l'intention du clinicien lorsque le processeur (120) détermine qu'aucune condition de filtre d'alerte ne contredit la décision de classification limite obtenue.

4. Méthode selon la revendication 1 dans laquelle au moins une condition du filtre d'alerte concerne au moins l'un des éléments suivants: l'historique des visites du patient, la réponse du patient à un traitement, un modèle de caractéristique mesurée cliniquement, un type de mesure clinique et un changement dans une caractéristique clinique mesurée.

5. Méthode selon la revendication 1 dans laquelle au moins un filtre d'alerte (165, 166) est sélectionné sur la base du modèle de classification (162, 163).

6. Méthode selon la revendication 1 dans laquelle les données contextuelles relatives au patient comprennent au moins l'humeur du patient, l'apparence physique du patient, l'état émotionnel du patient et l'agilité du patient.

7. Système (100) de gestion des alertes au cours d'au moins une évaluation, un diagnostic et un traitement du patient, le système comprenant: une interface (150) pour recevoir les données du patient; et un processeur (120) exécutant des instructions stockées dans une mémoire (130, 160) pour: obtenir une décision de classification à partir d'un classificateur exécutant un modèle de classification (162, 163), la décision de classification étant basée sur les données du patient, déterminer si la décision de classification est une décision de classification limite, appliquer au moins un filtre d'alerte (165, 166) aux données du patient après avoir déterminé que la décision de classification est une décision de classification limite, dans laquelle au moins un filtre d'alerte (165, 166) est appliqué pour déterminer s'il faut émettre une alerte de données contextuelles à un clinicien pour l'inciter à prendre en compte les données contextuelles relatives au patient, **caractérisé en ce que** le processeur applique au moins un filtre d'alerte (165, 166) en analysant les données du patient pour déterminer si au moins une condition du filtre d'alerte est en contradiction avec la décision de classification limite obtenue, et émettre, à l'aide d'une interface utilisateur (140), une alerte de données contextuelles au clinicien après avoir déterminé qu'une condition d'alerte contredit la décision de classification limite obtenue.

8. Système (100) selon la revendication 7 dans lequel le processeur (120) est en outre configuré pour déterminer si la décision de classification est une décision de classification limite en: comparant la décision de classification à une limite de décision, en déterminant si la décision de classification se situe dans un seuil prédéterminé par rapport à la limite de décision, et en déterminant que la décision de classification est une décision de classification limite après avoir déterminé que la décision de classification se situe à l'intérieur d'un seuil prédéterminé par rapport à la limite de décision.

9. Système (100) selon la revendication 7, dans lequel le processeur (120) n'émet aucune alerte de données contextuelles au clinicien après avoir déterminé qu'aucune condition de filtre d'alerte ne contredit la décision de classification limite obtenue.

10. Système (100) selon la revendication 7, dans lequel au moins une condition du filtre d'alerte concerne au moins l'un des éléments suivants: historique des visites du patient, réaction du patient à un traitement, schéma d'une caractéristique mesurée cliniquement, type de mesure clinique et modification d'une caractéristique clinique mesurée.

11. Système (100) selon la revendication 7 dans lequel l'au moins un filtre d'alerte est sélectionné sur la base du modèle de classification.

12. Système (100) selon la revendication 7 dans lequel les données contextuelles relatives au patient comprennent au moins l'humeur du patient, l'apparence physique du patient, l'état émotionnel du patient et l'agilité du patient.

13. Support lisible par ordinateur non transitoire contenant des instructions exécutables par ordinateur pour l'exécution d'une méthode de gestion des alertes au cours d'au moins une évaluation, un diagnostic et un traitement de patient, le support comprenant: Instructions exécutables par ordinateur pour la réception de données de patients à l'aide d'une interface (150); des instructions exécutables par ordinateur pour obtenir, au niveau d'un processeur (120), une décision de classification à partir d'un classificateur exécutant un modèle de classification (162, 163), la décision de classification étant basée sur les données du patient; des instructions exécutables par ordinateur (161) pour déterminer, à l'aide d'un processeur (120) exécutant des instructions stockées dans une mémoire (130, 160), si la décision de classification est une décision de classification limite; instructions exécutables par ordinateur pour appliquer, à l'aide du processeur (120), au moins un filtre d'alerte (165, 166) aux données du patient après avoir déterminé que la décision de classification est une décision de classification limite, dans laquelle au moins un filtre d'alerte (165, 166) est appliqué pour déterminer s'il faut émettre une alerte de données contextuelles à un clinicien pour l'inciter à prendre en compte les données contextuelles relatives au patient, **caractérisé par le fait que** l'application d'au moins un filtre d'alerte (165, 166) comprend l'analyse des données du patient pour déterminer si au moins une condition de filtre d'alerte contredit la décision de classification limite obtenue; et **caractérisé en outre en ce que** le support comprend des instructions exécutables par ordinateur pour émettre, à l'aide d'une interface utilisateur (140), une alerte de données contextuelles au clinicien lorsque le processeur (120) détermine qu'une condition de filtre d'alerte contredit la décision de classification limite obtenue.

FIG. 1

FIG. 2

EP 3 833 240 B1

FIG. 3

400

```
┌─────────────────────────┐
│ Select classification model │
│          410            │
└─────────────────────────┘
            │
            ▼
┌─────────────────────────┐
│ Apply classification model │
│          420            │
└─────────────────────────┘
            │
            ▼
        ◇─────────◇                    ┌─────────────────────────┐
       ╱           ╲         N          │  No contextual data alert │
      ◇  Borderline? ◇ ──────────────▶ │          440            │
       ╲    430     ╱                   └─────────────────────────┘
        ◇─────────◇                                ▲
            │ Y                                     │
            ▼                                       │
┌─────────────────────────┐                        │
│   Apply alert filter(s)  │                        │
│          450            │                        │
└─────────────────────────┘                        │
            │ Y                                     │
            ▼                                       │
        ◇─────────◇                                 │
       ╱   Alert    ╲          N                    │
      ◇ condition contradict ◇ ─────────────────────┘
       ╲ classification?    ╱
        ◇    460    ◇
            │
            ▼
┌─────────────────────────┐
│ Issue contextual data alert │
│          470            │
└─────────────────────────┘
```

# FIG. 4

500

Z-score

Max

Relative distance:
$R_1/R_2$

Slope

Current

$R_2$

Intercept

$R_1$

Min

-$t_i$

ED

Time

Disposition

## FIG. 5

600

Receiving patient data
602

Receiving a classification decision
604

Determining whether the classification
decision is a borderline classification decision
606

Applying at least one filter to the patient data
upon determining the classification decision
is a borderline classification decision
608

Issuing a contextual data alert
610

## FIG. 6

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20140155763 A1
- WO 2015173675 A1
- US 20160302671 A1
- US 2017098037 A1
- WO 2018099767 A1
- WO 2018102821 A1